# EUROPEAN PATENT SPECIFICATION

(11) **EP 2 966 162 B1**
(45) Date of publication and mention of the grant of the patent: **31.05.2017**
(21) Application number: 14849553.4
(22) Date of filing: 26.09.2014
(51) Int. Cl.: C12M 1/00, F24F 7/06, B25J 21/00

(54) **WORK CHAMBER**
ARBEITSRAUM
CHAMBRE DE TRAVAIL

(30) Priority: 30.09.2013 JP 2013203339
(43) Date of publication of application: 13.01.2016
(73) Proprietor: Panasonic Healthcare Holdings Co., Ltd., Tokyo 105-8433 (JP)
(72) Inventor: KOBAYASHI, Koichi, Toon-shi, Ehime 791-0395 (JP); YOKOI, Yasuhiko, Toon-shi, Ehime 791-0395 (JP)
(74) Representative: Grünecker Patent- und Rechtsanwälte PartG mbB
(86) International application number: PCT/JP2014/075624
(87) International publication number: WO 2015/046424

(56) References cited:
- WO-A1-2013/125374
- JP-A- 2011 177 091

## Description

### [Technical Field]

The present disclosure relates to a working chamber, such as an isolator, a clean bench, or a cabinet, used for a regenerative medical experiment environmental device and a pharmaceutical experiment environmental device.

### [Background]

In an embodiment 1, an isolator is disclosed that includes a working chamber for conducting work such as observation of cultured cells, for example.

### [Citation List]

### [Patent Literature]

[Patent Literature 1] Japanese Patent Application Laid-open Publication No. 2011-177091

Document WO 2013/125374 A1 discloses a working chamber according to the preamble of claim 1.

### [Summary]

### [Technical Problem]

It is the object of the present Invention to provide a working chamber that is capable of conducting observation in a work space and also improves workability.

### [Solution to Problem]

This object is solved by the features of claim 1. Further improvements are laid down in the subclaims. A working chamber according to the present disclosure includes: a box-shaped body case including a work space in an interior thereof and an insertion portion, in a front surface thereof, to which a glove for inserting a worker's arm is mounted; a supply device configured to supply gas in an exterior of the body case, through a supply filter, into the work space in the body case; a work plate configuring a bottom surface of the work space, the bottom surface having an opening formed therein; a storage chamber provided below the work plate, the storage chamber communicating with the work space through the opening; and an observation device capable of being lifted and lowered so that the whole thereof is disposed in the storage chamber when the device is in a housed state and at least a part thereof is disposed in the work space when the device is in an in-use state,
the observation device having a receiving portion on a side surface thereof, the receiving portion having a width in a horizontal direction greater than a gap between the work plate and the observation device in the in-use state,
the receiving portion being configured to close the gap between the observation device and the work plate, with a part of the receiving portion and a back surface of the work plate being in contact with or adjacent to each other, in the in-use state of the observation device.

### [Advantageous Effects]

A working chamber according to the present disclosure is capable of conducting observation in a work space and also is effective in improving workability.

### [Brief Description of the Drawings]

[Fig. 1] Fig. 1 is a front view illustrating an isolator system according to an embodiment 1.
[Fig. 2] Fig. 2 is a perspective view of the isolator system according to the embodiment 1.
[Fig. 3] Fig. 3 is a perspective view illustrating the isolator system when an incubator is mounted thereto according to the embodiment 1.
[Fig. 4] Fig. 4 is a top view illustrating a work plate according to the embodiment 1.
[Fig. 5] Fig. 5 is a perspective view illustrating an observation unit according to the embodiment 1.
[Fig. 6] Fig. 6 is a 5-5 cross-sectional view in Fig. 4 when the observation device according to the embodiment 1 is in a housed state.
[Fig. 7] Fig. 7 is a 5-5 cross-sectional view in Fig. 4 when the observation device according to the embodiment 1 is in an in-use state.

### [Mode for Carrying Out the Disclosure]

Hereinafter, embodiments will be described in detail with reference to drawings as necessary. However, a description in more detailed than necessary may be omitted. For example, detailed descriptions of well-known matters and the repeated descriptions of substantially the same configurations may be omitted. This is to prevent the following description from being unnecessarily redundant than necessary, and to facilitate the understanding of a person skilled in the art.

Note that the invertors provide the accompanying drawings and the following descriptions to help a person skilled in the art fully understand the present disclosure, and are not intended to limit the subject matters described in the claims thereby.

### (Embodiment 1)

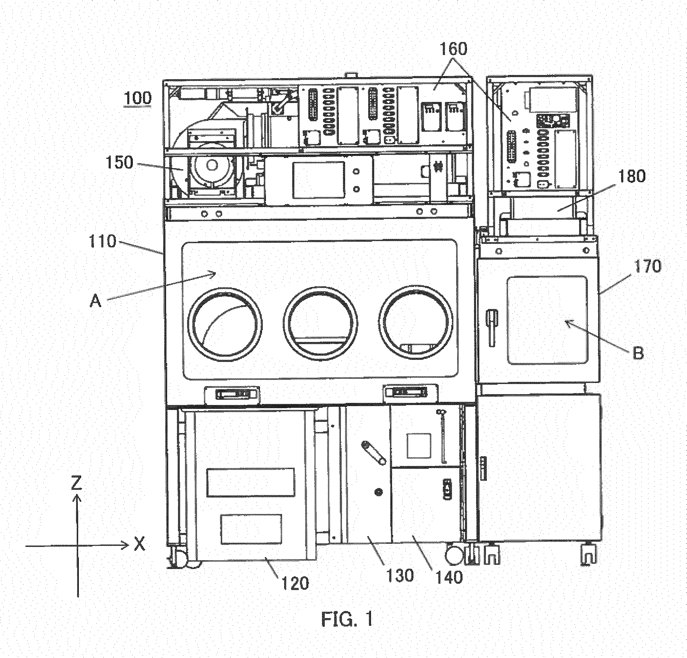

An isolator system 100 will be described hereinafter as an example of a working chamber in an embodiment 1 with reference to Figs. 1 to 7.

The isolator system 100 in the embodiment 1 is a device configured to perform, for example, work for cell culture, manipulation, observation, etc., in a sterilized environment. Note that sterilization means an act of killing microorganisms, cells and the like, to bring a state closer to a sterile environment.

Note that, in the present embodiment, it is assumed that the Z axis is an axis along a vertical direction in which the isolator system 100 is provided in a standing manner, and a direction toward the upper side is +Z direction and a direction toward the lower side (downward) is -Z direction. It is assumed that the Y axis is an axis along a direction perpendicular to the front surface and the back surface of the isolator system 100, and a direction from the front surface, where openings for conducting work in the interior of a work space are provided, toward the back surface opposite to the front surface is -Y direction, and a direction from the back surface toward the front surface is +Y direction. It is assumed that the X axis is an axis along a direction perpendicular to the left and right side surfaces when seen from the front, and a direction from the left side surface toward the right side surface when seen from the front is +X direction, and a direction from the right side surface toward the left side surface is -X direction.

### [1. Isolator System]

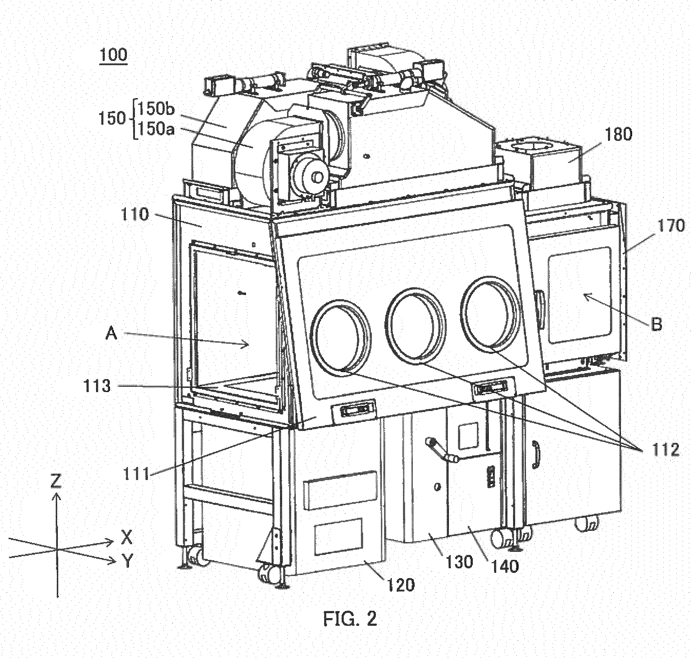

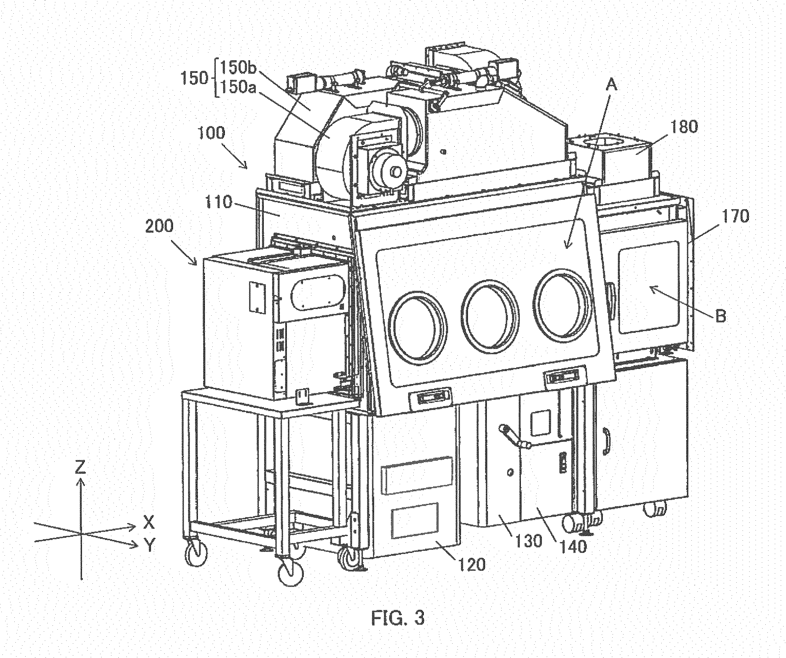

A configuration of the isolator system 100 will be described with reference to Figs. 1 to 3. Fig. 1 is a front view illustrating the isolator system 100 according to the embodiment 1. Fig. 2 is a perspective view illustrating the isolator system 100 according to the embodiment 1. Fig. 3 is a perspective view of the isolator system 100 when an incubator 200 is mounted thereto, according to the embodiment 1.

As illustrated in Fig. 1, the isolator system 100 according to the embodiment 1 includes: a glove box 110; a centrifuge unit 120; an observation unit 130; a sterilization unit 140; an air conditioning unit 150; a control unit 160; a pass box 170; and an air conditioning unit 180. The control unit 160 is provided above the glove box 110 and the pass box 170, and is configured to control the operations of the devices such as the sterilization unit 140 and the air conditioning unit 150.

As illustrated in Fig. 2, the glove box 110 includes a substantially box-shaped work space A, formed in the interior thereof, that is isolated from the surroundings to conduct work in the sterilized environment. The glove box 110 has a plurality of front surface openings 112, to which gloves (not shown) are mounted, in a front surface plate 111. The front surface plate 111 is openable/closable about a hinge provided an upper end thereof serving as an axis. Thus, the front surface of the glove box 110 can be opened/closed. Openings for mounting the pass box 170 and the incubator 200 are formed in left and right side surface plates. The pass box 170 is mounted to the opening in the right side surface plate of the glove box 110, and the incubator 200 is mounted to the opening in the right side surface plate thereof. A worker conducts work in the interior of a work space A through gloves at the time of working. The pass box 170 also includes a substantially box-shaped preparation space B, formed in the interior thereof, that is isolated from the surroundings to sterilize, in advance, items which are to be conveyed from the exterior into the work space A. The work space A and the preparation space B are isolated from each other via a door, and communicate with each other with the door being opened.

In the glove box 110, the box-shaped body case is configured with the front surface plate 111 having the plurality of front surface openings 112, which are insertion portions for worker's hands, a back surface plate, a top surface plate, a bottom surface plate, and left and right side surface plates. The glove box 110 includes, in the interior thereof: a work plate 113 on which work is conducted; and a divider provided on the back surface side of the glove box 110. The box shaped work space A that is a space for conducting work is formed with the front surface plate 111, the top surface plate, the work plate 113, the divider, and the left and right side surface plates. The glove box 110 is a compartment that is formed with airtightness so as to restrain bacterial invasion from the exterior. In the glove box 110, the work plate 113, the divider, the bottom surface plate, the back surface plate, the top surface plate, and the left and right side surface plates are configured with stainless steel plates, which is easily cleaned and sterilized. A bottom surface duct is formed between the work plate 113 and the bottom surface plate as a discharge path through which the gas in the work space A is discharged. Further, a back surface duct is formed between the divider and the back surface plate as a discharge path through which the gas in the work space A is discharged. The bottom surface duct and the back surface duct communicate with each other. The gas supplied into the glove box 110 passes through the bottom surface duct and the back surface duct, and is discharged from the glove box 110. An inlet and an outlet are provided in the top surface plate in the glove box 110. The inlet is disposed at the upper front side of the work space A and the outlet is disposed at the upper rear side thereof. The gas is supplied from the inlet into the work space A, and the gas in the work space A is discharged from the outlet. In the glove box 110, a particulate trap filter such as an HEPA filter is mounted to the inlet to secure the sterile environment in the interior, and the gas is supplied through the particulate trap filter into the glove box 110. Further, a particulate trap filter such as the HEPA filter is mounted to the outlet, and the gas in the glove box 110 is discharged from the interior of the glove box 110 through the particulate trap filter.

The centrifuge unit 120 is provided below the glove box 110, and can be connected from the work space A. The centrifuge unit 120 includes, in the interior thereof, a centrifuge configured to centrifuge a sample to work with in the work space A. The observation unit 130 is provided below the glove box 110, and can be connected from the work space A. The observation unit 130 includes, in the interior thereof, an observation device configured to observe a sample to work with in the work space A. Further, the observation unit 130 includes: a lifting mechanism capable of lifting and lowering the observation device provided in the interior thereof; and a handle, provided in the exterior, with which the lifting mechanism is operated. A worker operates the lifting mechanism using the handle, so that the observation device can be moved up into the work space A when the observation device is used and the observation device can be housed in the observation unit 130 when the observation unit 130 is not used. The sterilization unit 140 is provided below the glove box 110, to supply a sterilizing substance such as hydrogen peroxide into the glove box 110. The sterilization unit 140 in the present embodiment is configured to spray sterilizing mist, obtained by converting sterilizing liquid into mist, through a nozzle provided in the glove box 110, to sterilize the interior thereof.

The air conditioning unit 150 is provided above the glove box 110, and is configured to control the air conditioning in the interior thereof. The air conditioning unit 150 includes a supply unit 150a and a discharge unit 150b. The air conditioning unit 150 is configured to supply outside air into the work space A using the supply unit 150a, and discharge the gas in the work space A using the discharge unit 150b. The air conditioning unit 180 is provided above the pass box 170, and is configured to control the air conditioning in the preparation space B in the pass box 170.

As illustrated in Fig. 2, in the isolator system 100, the incubator 200 is mounted to the side surface opposite to the side surface to which the pass box 170 is provided in the glove box 110. The incubator 200 includes a culture chamber (not shown) in the interior thereof. The culture chamber is a chamber for storing a culture and performing culture in the interior thereof, and is partitioned as a space to restrain bacterial invasion from the exterior, for example, by a rectangular parallelepiped-shaped box body. The culture chamber is partitioned with, for example, stainless steel plates. The incubator 200 is demountably configured with respect to the isolator system 100. Thus, culture can be controlled in each incubator 200. For example, a dedicated incubator 200 can be used for each donor, thereby restraining occurrence of failures such as mix-up of culture.

### [2. Work Plate]

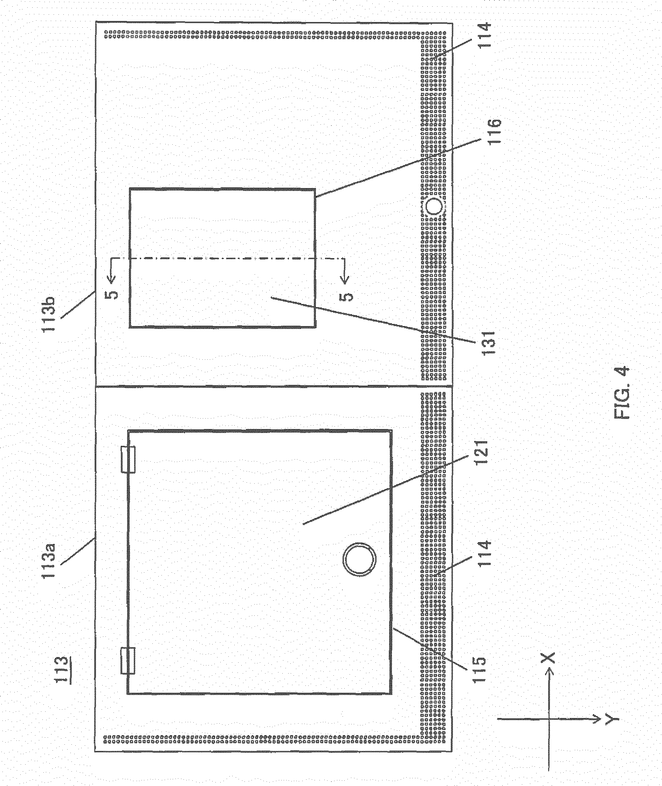

A configuration of the work plate 113 will be described with reference to Fig. 4. Fig. 4 is a top view illustrating the work plate 113 according to the embodiment 1.

As illustrated in Fig. 4, the work plate 113 is configured with a first work plate 113a and a second work plate 113b obtained by dividing the work plate into left and right. The work plate 113 has a plurality of outlets 114, through which the gas in the work space A is discharged, formed in the front end portion and left and right end portions. A connection opening 115 for connecting to the centrifuge unit 120, provided below the work plate 113, is formed in the first work plate 113a. A centrifuge cover 121, which configures a part of the first work plate 113a and is openable and closable with respect to the connection opening 115, is mounted via a hinge to the first work plate 113a. A connection opening 116 for connecting to the observation unit 130, provided below the work plate 113, is formed in the second work plate 113b. The connection opening 116 is covered with an observation device cover 131, and the observation device cover 131 is disposed in the same plane as the second work plate 113b.

### [3. Observation Unit]

### [3-1. Configuration]

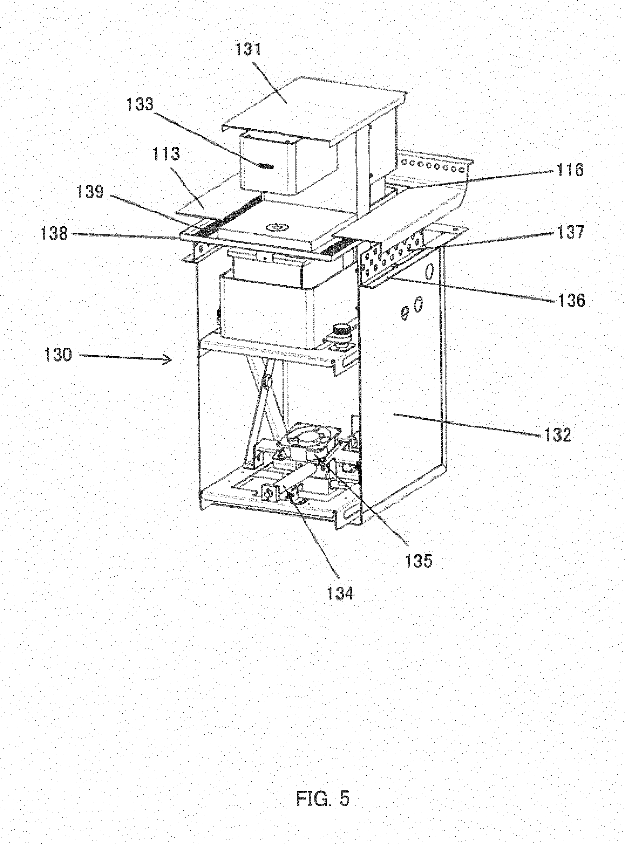

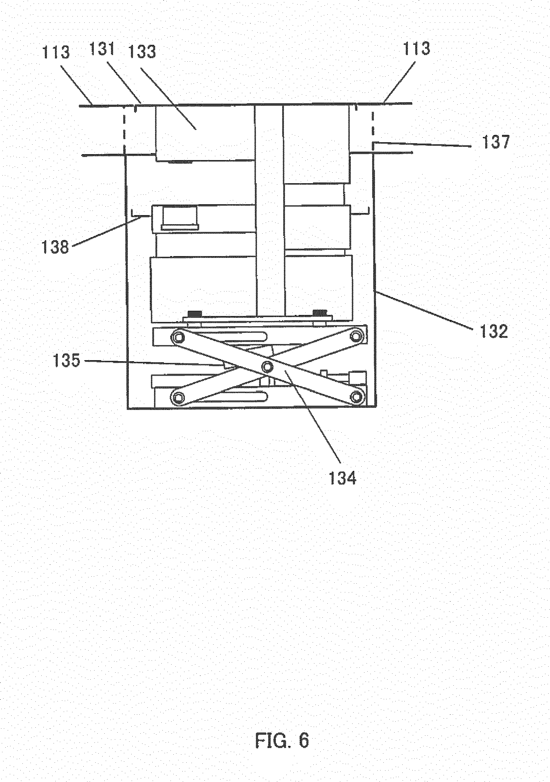

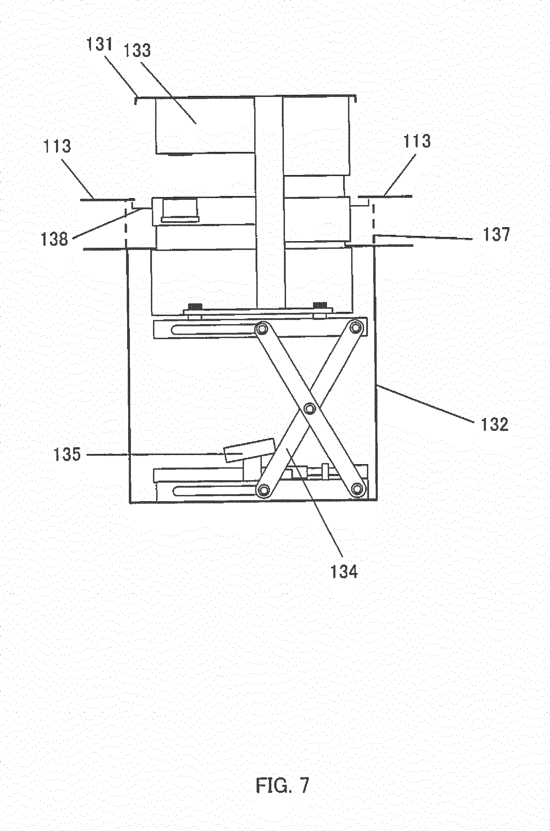

A configuration of the observation unit 130 will be described with reference to Fig. 5. Fig. 5 is a perspective view illustrating the observation unit 130 according to the embodiment 1. Fig. 6 is a 5-5 cross-sectional view in Fig. 4 when an observation device 133 according to the embodiment 1 is in a housed state, and Fig. 7 is a 5-5 cross-sectional view in Fig. 4 when in an in-use state.

The observation unit 130 includes: a storage chamber 132; the observation device 133; a lifting device 134; and a fan 135. The storage chamber 132 houses the observation device 133 inside. The storage chamber 132 is provided below the work plate 113, and communicates with the work space A through the connection opening 116. Specifically, the storage chamber 132 is a box shaped case whose top face is opened, and the edge of such opening is provided with a flange 136 extending outward. An opening for fitting the storage chamber 132 is formed in the bottom surface plate in the glove box 110, and the storage chamber 132 is fit into the opening from above. Then, the flange 136 of the storage chamber 132 is mounted to the bottom surface plate of the glove box 110, and thus the work space A and the storage chamber 132 communicate with each other. Further, a regulating plate 137 in a frame shape extending upward is provided to the opening edge of the top face of the storage chamber 132. The regulating plate 137 is provided between the bottom surface plate of the glove box 110 and the work plate 113, that is, in the bottom surface duct. Thus, in the regulating plate 137, a plurality of hole portions are formed so that gasified sterilizing substance can pass therethrough. As a result, an instrument or liquid that has fallen from the plurality of outlets 114 of the work plate 113 to the bottom surface duct is prevented from further falling into the storage chamber 132.

In the interior of the storage chamber 132, the lifting device 134 capable of being operated from the exterior is provided, and the observation device 133 is fixed to the upper part of the lifting device 134. The lifting device 134 can be operated from the exterior by a worker, to raise or lower the observation device 133. The whole of the observation device 133 is disposed in the storage chamber 13 when the observation device is in the housed state, and the upper portion of the observation device 133 is disposed in the work space A when the observation device is in the in-use state in which the observation device 133 can be used. In the present embodiment, in the in-use state, a stage of the observation device 133 where an observation target is placed is positioned substantially in the same plane as the work plate 113.

Further, as described above, the upper surface of the observation device 133 is provided with the observation device cover 131 having the area slightly smaller than the area of the connection opening 116 of the work plate 113, and the observation device cover 131 is substantially in the same plane as the work plate 113 and also positioned within the connection opening 116 of the work plate 113 , in the housed state of the observation device 133.

Further, the observation device 133 includes a falling-object receiving portion 138, provided in such a manner as to surround the side surface thereof, to catch an instrument or the like that falls from a gap between the connection opening 116 of the work plate 113 and the observation device 133. The falling-object receiving portion 138 is made of metal plate such as stainless steel. The falling-object receiving portion 138 is in a shape extending outward from the side surface and then being bent upward, so as not to let such an instrument having been caught once further fall, and an upper end portion thereof is positioned above an inner end portion thereof. As illustrated in Fig. 6, the falling-object receiving portion 138 is formed such that the width thereof in the horizontal direction is greater than the gap formed between the connection opening 116 of the work plate 113 and the observation device 133 in the in-use state. Further, as illustrated in Fig. 7, in the falling-object receiving portion 138, under the in-use state of the observation device 133, the outer end of the falling-object receiving portion 138 and the back surface of the work plate 113 are in contact with or adjacent to each other, to close the gap between the connection opening 116 of the work plate 113 and the observation device 133. Note that being adjacent to each other means forming such a gap that the falling object having caught once by the falling-object receiving portion 138 does not fall outward from the gap (Z axis direction) between the falling-object receiving portion 138 and the back surface of the work plate 113, and it is desirable that the gap is smaller than the gap (XY axis direction) between the observation device 133 and the work plate 113. Further, in the falling-object receiving portion 138, a plurality of hole portions 139 are formed so that the gasified sterilizing substance can pass therethrough.

Further, the fan 135 configured to diffuse the gas in the storage chamber 132 is provided in the interior of the storage chamber 132.

### [4. Effects, etc.]

As described above, in the present embodiment, the isolator system 100 includes: the box shaped glove box 110 (body case) having the work space A in the interior thereof; and the front surface openings 112 (insertion portions) in the front surface plate thereof, to which gloves for inserting a worker's arms are mounted; the supply unit 150a (supply device) configured to supply the gas in the exterior of the glove box 110 through the particulate trap filter (supply filter) into the work space A in the glove box 110; the work plate 113 configuring the bottom surface of the work space A, the bottom surface having the connection opening 116 formed therein; the storage chamber 132 provided below the work plate 113, the storage chamber communicating with the work space A through the connection opening 116; and the observation device 133 capable of being lifted and lowered so that the whole thereof is disposed in the storage chamber 132 in the housed state, and at least a part thereof is disposed in the work space A in the in-use state. The observation device 133 has the falling-object receiving portion 138 (receiving portion) on the side surface thereof, the falling-object receiving portion having a width in the horizontal direction greater than the gap between the connection opening 116 of the work plate 113 and the observation device 133 in the in-use state. The falling-object receiving portion 138 is configured to close the gap between the connection opening 116 of the observation device 133 and the work plate 113, with the upper end portion of the falling-object receiving portion 138 and the back surface of the work plate 113 being in contact with or adjacent to each other, in the in-use state of the observation device 133. Thus, with the present disclosed technique, the isolator system 100 that is capable of conducting observation in the work space A and is also effective to improve workability, can be provided.

Further, the isolator system 100 further includes: the discharge unit 150b (discharge device) configured to discharge the gas in the work space A through the particulate trap filter (outlet filter) to the exterior of the body case; and the sterilization unit 140 (sterilization device) configured to supply a sterilizing substance into the work space A, and the falling-object receiving portion 138 of the observation device 133 has the plurality of hole portions 139 penetrating therethrough. Thus, the isolator system 100 can reliably supply the gasified sterilizing substance to the interior of the storage chamber 132.

Further, in the observation device 133, when the stage thereof where an observation target is placed is positioned substantially in the same plane as the work plate 113, the upper end portion of the falling-object receiving portion 138 and the back surface of the work plate 113 are in contact with or adjacent to each other. Thus, in the isolator system 100, since the work plate 113 on which work is conducted and the stage of the observation device 133 are substantially flush with each other in the in-use state, not only workability when conducting observation is improved, but also an instrument or the like is prevented from falling from the gap between the observation device 133 and the work plate 113.

Further, the falling-object receiving portion 138 extends outward from the inner end portion, positioned below the stage, on the side surface of the observation device 133, and the upper end portion of the falling-object receiving portion 138 is positioned above the inner end portion and below the stage. Thus, the isolator system 100 can reliably catch the falling object that is falling from the gap between the observation device 133 and the work plate 113.

Further, the observation device cover 131 (top board) having the area slightly smaller than the area of the connection opening 116 in the work plate 113 is provided on the upper surface of the observation device 133, and when the observation device 133 is in the housed state, the observation device cover 131 is substantially in the same plane as the work plate 113, and also is positioned inside the connection opening 116 of the work plate 113. Thus, since the observation device cover 131 becomes a part of the work plate 113 in the state where the observation device 133 is stored, workability on the work plate 113 is improved.

### (Other Embodiment)

As described above, the embodiment 1 has been described as an example of the technique disclosed in the present application. However, the present disclosed technique is not limited thereto, but is applicable to an embodiment in which modifications, replacements, additions, omissions and the like are made as appropriate. Further, the components described in the above embodiment 1 can be combined to configure a new embodiment.

Then, other embodiments will hereinafter be exemplified. Note that the same components as those in the embodiment 1 will be designated by the same reference numerals as those in the embodiment 1, for convenience' sake.

In the embodiment 1, the glove box 110 has been described as an example of the body case. The glove box 110 is not limited to a body case for an isolator in which work is conducted in the work space A through gloves. The present disclosed technique is useful also for a clean bench or a cabinet having the front surface openings 112 to which no glove is mounted. In short, the body case may be the one in which gas is supplied into the work space A to adjust an environment of the work space A, in an experimental environment device in which a worker inserts his/her hand into the work space A to conduct work in the interior thereof.

Further, in the embodiment 1, the falling-object receiving portion 138 has been described as an example of the receiving portion. As illustrated in Figs. 5 to 7, the falling-object receiving portion 138 is in such a shape as to extend outward from the side surface of the observation device and thereafter be bent upward. However, the receiving portion is not limited to this shape. The falling-object receiving portion 138 may be configured to have an inclined surface that is inclined upward and outward from the inner end portion. In that case, the fallen object that has fallen to the falling-object receiving portion 138 bounces or rolls not outward but inward. Thus, not only the fallen object is restrained from falling down outside the falling-object receiving portion 138, but also the fallen object is easily picked up. Further, the falling-object receiving portion 138 is made of metal plate such as stainless steel, and has the plurality of hole portions 139. However, the receiving portion is not limited to metal plate. The receiving portion may be configured with, for example, a metal mesh. In that case, since the receiving portion can be allowed to have elasticity, not only the falling object is restrained from bouncing when being caught thereby, but also a load that is applied to each other between the receiving portion and the back surface of the work plate 113 can be reduced when they are in contact with each other.

### [Industrial Applicability]

The present disclosure is applicable to an experimental environment device in which a work hand is inserted into a box shaped work space to conduct work.

### [Reference Signs List]

- 100: isolator system
- 110: glove box
- 111: front surface plate
- 112: front surface opening
- 113: work plate
- 113a: first work plate
- 113b: second work plate
- 114: a plurality of outlets
- 115: connection opening
- 116: connection opening
- 120: centrifuge unit
- 121: centrifuge cover
- 130: observation unit
- 131: observation device cover
- 132: storage chamber
- 133: observation device
- 134: lifting device
- 135: fan
- 136: flange
- 137: regulating plate
- 138: falling-object receiving portion
- 139: a plurality of hole portions
- 140: sterilization unit
- 150: air conditioning unit
- 150a: supply unit
- 150b: discharge unit
- 160: control unit
- 170: pass box
- 180: air conditioning unit
- 200: incubator

## Claims

1. A working chamber comprising:
a box-shaped body case including a work space (A) in an interior thereof and an insertion portion, in a front surface thereof;
a supply device configured to supply gas in an exterior of the body case, through a supply filter, into the work space (A) in the body case;
a discharge device (150b) configured to discharge gas in the work space (A), through an outlet filter, to the exterior of the body case;
a work plate (113) configuring a bottom surface of the work space (A), the bottom surface having an opening (116) formed therein;
a storage chamber (132) provided below the work plate (113), the storage chamber (132) communicating with the work space (A) through the opening (116); and
an observation device (133) capable of being lifted and lowered so that the whole thereof is disposed in the storage chamber (A) when the device (133) is in a housed state and at least a part thereof is disposed in the work space (A) when the device (133) is in an in-use state,
**characterized in that**
the observation device (133) having a receiving portion (138) on a side surface thereof, the receiving portion (138) having a width in a horizontal direction greater than a gap between the work plate and the observation device (133) in the in-use state,
the receiving portion (138) being configured to close the gap between the observation device (133) and the work plate (113), with an upper end portion of the receiving portion (138) and a back surface of the work plate (113) being in contact with or adjacent to each other, in the in-use state of the observation device (133).

2. The working chamber according to claim 1, further comprising:
a sterilization device configured to supply a sterilizing substance into the work space (A), wherein
the receiving portion (138) has a plurality of hole portions (139) penetrating therethrough.

3. The working chamber according to claim 1 or 2, wherein
in the observation device (133), when a stage thereof where an observation target is placed is positioned substantially in a same plane as the work plate (113), the upper end portion of the receiving portion (138) and the back surface of the work plate (113) are in contact with or adjacent to each other.

4. The working chamber according to claim 3, wherein
the receiving portion (138) extends outward from an inner end portion thereof, positioned below the stage, on the side surface of the observation device (133), and the upper end portion of the receiving portion (138) is positioned above the inner end portion and below the stage.

5. The working chamber according to claim 1, wherein
a top board (131) having an area slightly smaller than an area of the opening of the work plate (113) is provided on an upper surface of the observation device (133), and
the top board (131) is positioned substantially in a same plane as the work plate (113) and positioned within the opening (116) of the work plate (113), when the observation device (133) is in the housed state.

6. The working chamber according to claim 1, wherein
the receiving portion (138) has an inclined surface that is inclined upward and outward from an inner end portion.

## Patentansprüche

1. Arbeitsraum, umfassend:
ein kastenförmiges Körpergehäuse mit einem Arbeitsraum (A) im Inneren und einem Einführungsabschnitt in einer Frontfläche davon;
eine Zufuhrvorrichtung, die dazu konfiguriert ist, Gas von außerhalb des Körpergehäuses durch einen Zufuhrfilter in den Arbeitsraum (A) in dem Körpergehäuse einzuleiten;
eine Ausstoßvorrichtung (150b), die dazu konfiguriert ist, Gas in dem Arbeitsraum (A) durch einen Auslassfilter nach außerhalb des Körpergehäuses auszustoßen;
eine Arbeitsplatte (113), die eine Bodenfläche des Arbeitsraums (A) bildet, wobei in der Bodenfläche eine Öffnung (116) ausgebildet ist;
einen Speicherraum (132), der unterhalb der Arbeitsplatte (113) angeordnet ist, wobei der Speicherraum (132) durch die Öffnung (116) mit dem Arbeitsraum (A) in Verbindung steht; und
eine Beobachtungsvorrichtung (133), die dazu in der Lage ist, angehoben und abgesenkt zu werden, so dass sie im Ganzen in dem Speicherraum (A) angeordnet ist, wenn sich die Vorrichtung (133) in einem aufgenommenem Zustand befindet, und zumindest ein Teil davon in dem Arbeitsraum (A) angeordnet ist, wenn sich die Vorrichtung (133) in einem Benutzungszustand befindet,
**dadurch gekennzeichnet, dass**
die Beobachtungsvorrichtung (133) auf einer Seitenfläche einen Aufnahmeabschnitt (138) aufweist, wobei der Aufnahmeabschnitt (138) in einer horizontalen Richtung eine größere Breite als ein Spalt zwischen der Arbeitsplatte und der Beobachtungsvorrichtung (133) in dem Benutzungszustand aufweist,
der Aufnahmeabschnitt (138) so konfiguriert ist, dass er den Spalt zwischen der Beobachtungsvorrichtung (133) und der Arbeitsplatte (113) schließt, wobei in dem Benutzungszustand der Beobachtungsvorrichtung (133) ein oberer Endabschnitt des Aufnahmeabschnitts (138) und eine Rückseite der Arbeitsplatte (113) miteinander in Kontakt stehen oder sich nebeneinander befinden.

2. Arbeitsraum nach Anspruch 1, des Weiteren umfassend:
eine Sterilisationsvorrichtung, die dazu konfiguriert ist, eine sterilisierende Substanz in den Arbeitsraum (A) einzuleiten, wobei
der Aufnahmeabschnitt (138) eine Vielzahl von Lochabschnitten (139) aufweist, die ihn durchdringen.

3. Arbeitsraum nach Anspruch 1 oder 2, wobei,
wenn bei der Beobachtungsvorrichtung (133) ein Tisch, auf dem ein Beobachtungsziel platziert ist, im Wesentlichen in derselben Ebene wie die Arbeitsplatte (113) positioniert ist, der obere Endabschnitt des Aufnahmeabschnitts (138) und die Rückseite der Arbeitsplatte (113) miteinander in Kontakt stehen oder sich neben einander befinden.

4. Arbeitsraum nach Anspruch. 3, wobei
sich der Aufnahmeabschnitt (138) von einem inneren Endabschnitt, der unterhalb des Tisches auf der Seitenfläche der Beobachtungsvorrichtung (133) positioniert ist, nach außen erstreckt, und der obere Endabschnitt des Aufnahmeabschnitts (138) oberhalb des inneren Endabschnitts und unterhalb des Tisches positioniert ist.

5. Arbeitsraum nach Anspruch 1, wobei
eine obere Platte (131) mit einer geringfügig kleineren Fläche als eine Fläche der Öffnung der Arbeitsplatte (113) auf einer Oberseite der Beobachtungsvorrichtung (133) angeordnet ist, und die obere Platte (131) im Wesentlichen in derselben Ebene wie die Arbeitsplatte (113) positioniert und innerhalb der Öffnung (116) der Arbeitsplatte (113) positioniert ist, wenn sich die Beobachtungsvorrichtung (133) in dem aufgenommenen Zustand befindet.

6. Arbeitsraum nach Anspruch 1, wobei
der Aufnahmeabschnitt (138) eine geneigte Fläche aufweist, die von einem inneren Endabschnitt nach oben und außen geneigt ist.

## Revendications

1. Chambre de travail comprenant :
un châssis principal en forme de boîte incluant un espace de travail (A) dans un intérieur de celui-ci et une partie d'insertion, dans une surface avant de celui-ci ;
un dispositif d'alimentation configuré pour fournir du gaz situé à l'extérieur du châssis principal, au travers d'un filtre d'alimentation, dans l'espace de travail (A) du châssis principal ;
un dispositif d'évacuation (150b) configuré pour évacuer du gaz situé dans l'espace de travail (A), à travers un filtre de sortie, vers l'extérieur du châssis principal ;
un plan de travail (113) configurant une surface de fond de l'espace de travail (A), la surface de fond ayant une ouverture (116) formée dans celle-ci ;
une chambre de stockage (132) située en dessous du plan de travail (113), la chambre de stockage (132) communiquant avec l'espace de travail (A) à travers l'ouverture (116) ; et
un dispositif d'observation (133) pouvant être levé et abaissé de façon qu'il soit disposé en totalité dans la chambre de stockage (A) lorsque le dispositif (133) est dans un état rangé et qu'au moins une partie de celui-ci soit disposée dans l'espace de travail lorsque le dispositif est dans un état d'utilisation,
**caractérisée en ce que**
le dispositif d'observation (133) a une partie de réception (138) sur une surface latérale de celui-ci, la partie de réception (138) ayant une largeur dans une direction horizontale supérieure à un espace entre le plan de travail et le dispositif d'observation (133) dans l'état d'utilisation,
la partie de réception (138) est configurée pour fermer l'espace entre le dispositif d'observation (133) et le plan de travail (113), une partie d'extrémité supérieure de la partie de réception (138) et une surface arrière du plan de travail (113) étant en contact avec ou adjacente l'une à l'autre, dans l'état d'utilisation du dispositif d'observation (133).

2. Chambre de travail selon la revendication 1, comprenant en outre :
un dispositif de stérilisation configuré pour fournir une substance stérilisante dans l'espace de travail (A),
la partie de réception (138) ayant une pluralité de parties perforées (139) pénétrant à travers celle-ci.

3. Chambre de travail selon la revendication 1 ou 2, où
dans le dispositif d'observation (133), lorsqu'une plateforme de celui-ci sur laquelle est placée une cible à observer est positionnée sensiblement dans un même plan que le plan de travail (113), la partie d'extrémité supérieure de la partie de réception (138) et la surface arrière du plan de travail (113) sont en contact ou adjacentes l'une à l'autre.

4. Chambre de travail selon la revendication 3, dans laquelle
la partie de réception (138) s'étend vers l'extérieur depuis une partie d'extrémité intérieure de celle-ci, positionnée sous la plateforme, sur la surface latérale du dispositif d'observation (133), et la partie d'extrémité supérieure de la partie de réception (138) est positionnée au-dessus de la partie d'extrémité intérieure et en dessous de la plateforme.

5. Chambre de travail selon la revendication 1, dans laquelle
une plaque de dessus (131) ayant une aire légèrement inférieure à une aire de l'ouverture du plan de travail (113) est située sur une surface supérieure du dispositif d'observation (133), et la plaque de dessus (131) est positionnée sensiblement dans un même plan que le plan de travail (113) et positionnée à l'intérieur de l'ouverture (116) du plan de travail (113), lorsque le dispositif d'observation est dans l'état rangé.

6. Chambre de travail selon la revendication 1, dans laquelle
la partie de réception (138) a une surface inclinée qui est inclinée vers le haut et vers l'extérieur à partir d'une partie d'extrémité intérieure.
